# EUROPEAN PATENT APPLICATION

(11) **EP 1 241 477 A1**
(43) Date of publication of application: **18.09.2002**
(21) Application number: 01200929.6
(22) Date of filing: 12.03.2001
(51) Int. Cl.: G01N 33/569, C12Q 1/10, C07K 14/315, C12N 9/88, C12N 15/31, C12N 15/60

(54) **Diagnosis and treatment of enterococcus faecium related disease**

(71) Applicant: Universitair Medisch Centrum Utrecht, 3584 CX Utrecht (NL); Universiteit Utrecht, 3584 CG Utrecht (NL)
(72) Inventor: Willems, Robertus J. L., 3993 HK Houten (NL); Bonten, Marcus J. M., 3584 CX Utrecht (NL)
(74) Representative: Prins, Adrianus Willem

(57) **Abstract**

The invention provides a method for determining whether an *E. faecium* bacterium is epidemic, comprising determining whether an epidemic *E. faecium* nucleic acid sequence and/or an expression product thereof is present in said bacterium, and/or associated with said bacterium. The invention further provides a method for diagnosis of an epidemic *E. faecium* related disease in a patient, comprising:
- obtaining a sample from said patient, and
- detecting the presence of an *E. faecium esp* gene and/or an epidemic *purK* housekeeping gene or a functional equivalent thereof, and/or an expression product of any of said genes or a functional part, derivative and/or analogue of said product in said sample. The invention also provides an isolated or recombinant *E. faecium esp* expression product or a functional part, derivative and/or analogue thereof. In addition the invention provides an isolated or recombinant expression product of an epidemic *purK* housekeeping gene or a functional equivalent of said gene, or a functional part, derivative and/or analogue of said product. Also included is a method for treatment of an *E*. *faecium* related disease, comprising at least in part decreasing the attachment of an *E. faecium* bacterium to a host cell.

SEQUENCE LISTING

<110> Universitair Medisch Centrum Utrecht

<120> Diagnosis and treatment of Enterococcus faecium related
      disease

<130> P55610EP00

<140> 01200929.6
<141> 2001-03-12

<160> 6

<170> PatentIn Ver. 2.1

<210> 1
<211> 21
<212> DNA
<213> Artificial Sequence

<220>
<223> Description of Artificial Sequence: primer

<220>
<221> misc_feature
<222> (1)..(21)

<400> 1
gcagattggc acattgaaag t                                                21


<210> 2
<211> 21
<212> DNA
<213> Artificial Sequence

<220>
<223> Description of Artificial Sequence: primer

<220>
<221> misc_feature
<222> (1)..(21)
<223> /note="Y on position 21 can be C or T"

<400> 2
tacataaatc ccgcctgttt y                                                21


<210> 3
<211> 22
<212> DNA
<213> Artificial Sequence

<220>
<223> Description of Artificial Sequence: primer Esp 11

<220>
<221> misc_feature
<222> (1)..(22)

<400> 3
ttgctaatgc tagtccacga cc                                                22

<210> 4
<211> 23
<212> DNA
<213> Artificial Sequence

<220>
<223> Description of Artificial Sequence: primer Esp 12

<220>
<221> misc_feature
<222> (1)..(23)

<400> 4
gcgtcaacac ttgcattgcc gaa                                               23

<210> 5
<211> 890
<212> DNA
<213> Enterococcus faecium

<220>
<221> gene
<222> (1)..(890)
<223> /note="Enterococcus faecium esp gene"

<400> 5
gatatgtttt acaattgact aagccattag atgaaatctt agaagatatg aaagcaacag 60
cacaagttga agaaaataaa ccatttggtg aagatttcat ctttgattct tggttgtcgg 120
atacgaataa aaaattaatc cagaacactt atggaacagg ttattattat ttgcaagata 180
ttgatggtga tggaaaccct gacgataaag aagagagcgg agacacgaat ccatatatcg 240
ggaaacctga attagaagaa gtatatgatg ttgacacaac agttaagggg aaagtattca 300
tccacgagtt agcgggaaca ggtcacaaag cccaacttgt tgataaagaa ggtactgtat 360
tagcagaaaa aactatcgct ccaaatgaaa aagatggggc tccaatttca gatactgtag 420
aatttgaatt tacgggtgta gattcaagta aattaatcgc gaaagatgaa ttaaaaatcc 480
aaatcgtttc tccaggtttt gataaaccag aagaaggttc aaccgttatt aaggaatcac 540
caaaagcggt tgataaacaa accgtggtag ttggatttaa accagatgct aaagaatcaa 600
ttcggaataa taaaaactta cctgaagatg cagagtattc atggaaaaca gagcctgata 660
cttctaacgt tactgatagt acgaaaggta ttgtaactgt taagatcgga aatcgaactt 720
tcgacgtgga tgtagagttt gctgtaaaag cttctcaagc tatggaaaat gatgcaacat 780
acgtacctat aacaacaact ccagaaacga cagttcaaag tggaaaacct acatttgata 840
aaccagatgt tcctttagct aaagatgcct tttcaatttc agatgtctat            890

<210> 6
<211> 492
<212> DNA
<213> Enterococcus faecium

<220>
<221> allele
<222> (1)..(492)
<223> /note="Enterococcus faecium purk gene allele-1"

<400> 6
cgtcagtgta gaggcgttaa atgcgatttt acccatgtca tttattcctc aagggacaga 60
cttgcttgcg attacgcaag accgcctgtt agaaaaatct tttttggaaa cgaataacat 120

cgtgattgct ccttatgcaa cgatcgtcag tccgacggat atccaagatg cgattgacgg 180
gatcggctat ccttgtgttt tgaaaaccac tcgaggcggc tatgatggca aggggcagta 240
tgttttgaaa agtcgggcgg atctagctcc tgccatggac cttttaagag aaggaacctg 300
tgttttagaa gcatggatcc cgtttgaaaa agagatttcg atcatggtgg caggaaatgg 360
tcaaggagat ttcacgactt ttccggtcgt ggaaaacatc caccacaaca atattcttca 420
tgaaacgatt gctccagccg cgattgatca ggatgtgatc gaggaggctg agcgtattgc 480
ccgtgtgatt gc 492

## Description

The invention relates to the field of medicine. More specifically, the invention relates to the detection of epidemic *E. faecium,* and treatment of *E. faecium* related diseases.

The emergence of antibiotic resistance has escalated dramatically in recent years and now represents one of the most threatening public health problems (1,2). Among nosocomial pathogens, the emergence of *vanA*-containing vancomycin-resistant *Enterococcus faecium* (VREF) is of great concern. VREF are resistant to almost all commercially available antimicrobial agents, and their spread faces hospitals with the prospect of a post-antibiotic era. In the United States, the proportion of VREF among enterococci isolated from blood cultures increased from 0% in 1989 to 25.9% in 1999 (3).

There is a striking difference in the epidemiology of VREF between the U.S. and Europe. In the U.S., colonization with VREF has become endemic in many hospitals (4), whereas in Europe, intestinal colonization with VREF is common among healthy subjects and livestock. This community reservoir is large, with 2-15% of healthy subjects being colonized (5-12). Despite this large community reservoir and despite the presumed European origin of VREF, hospital outbreaks still occur only sporadically on this continent, even in settings with high selective antibiotic pressure, such as intensive care and haemodialysis units (13, 14). The epidemiological difference between the U.S. and Europe may partly be explained by differences in glycopeptide consumption between the two continents. In the U.S., vancomycin use in hospitals has increased more than 100-fold over the past 20 years, and is now quadrupling vancomycin use in European countries (15). This probably contributed to the rapid intra-hospital spread of VREF in the U.S. The presence of a community reservoir of VREF in Europe has been associated with the massive use of avoparcin, a glycopeptide antibiotic, as growth promoter in the veterinary industry between the early 1970s and 1997. Avoparcin has never been licensed in the U.S., which may explain the virtual absence of VRE in healthy humans and in farm animals (16-20). The presumed association between high carriage rates of VREF among livestock and colonization of healthy individuals prompted the European Union to ban the use of avoparcine for growth promotion in 1997. Since then, the prevalence of VREF colonization in the community has declined in several European countries (21-24). In Australia the repeated isolation of VREF from hospitalized patients first occurred in 1996, and VREF have an epidemiology which is different from that in Europe or the U.S., with *vanB E. faecium* being most prevalent (25). The predominant role of antibiotic pressure in the epidemiology of VREF was challenged by the recent occurrence of three outbreaks of VREF in The Netherlands, a country with, together with Scandinavian countries, the lowest antibiotic use in the developed world (26).

We have demonstrated the existance of epidemic and non-epidemic VREF strains. The observed difference in epidemic VREF, which can spread rapidly and are capable of causing disease in an individual, and non-epidemic VREF isolates which can hardly spread, reflected differences in the presence of virulence traits, but little was known about virulence factors in *E. faecium* (34). Knowledge about epidemic VREF is highly desirable, because a risk of hospital outbreaks is present each time a new patient is brought into a hospital. Bacteria of said new patient might colonize other patients. Simple determination of the presence of an *E. faecium* strain in said patient does not determine an epidemic *E. faecium* related disease, because many healthy subjects are colonized with non-epidemic *E. faecium,* especially in Europe. Until the present invention, there was no suitable method to distinguish between epidemic and non-epidemic *E. faecium.* Because of its resistance to antibiotics, it is very important to avoid hospital outbreak of VREF. The risk of outbreaks could be lowered by isolating every new patient. Only after two days it would be clear whether a patient is colonized with epidemic VREF. In practice, it is not convenient to isolate every patient until it is clear whether said patient is colonized with epidemic VREF or not. A suitable method to distinguish between epidemic and non-epidemic *E. faecium* would be highly desirable to identify patients carrying epidemic VREF. Once these patients would have been identified, they would be isolated to avoid hospital outbreaks, whereas patients carrying non-epidemic VREF would not have to be isolated. Precautions would not be necessary if a patient was known to carry only non-epidemic strains of *E. faecium*.

Thus, there was a need of a method to determine whether a certain *E. faecium* strain is well capable of transmission, i.e. can spread rapidly. More specifically, there was a need of a diagnostic method to determine whether a patient suffers from an epidemic *E. faecium* related disease. As already mentioned above, simple determination of the presence of an *E. faecium* strain in said patient does not distinguish between epidemic and non-epidemic *E. faecium.*

We have demonstrated a genetic relationship between epidemic hospital isolates of VREF from three different continents and a genetic distinction between these epidemic isolates and the non-epidemic VREF isolated from humans and animals in Europe. The presence of the enterococcal surface protein *(esp)* gene and a specific sequence of the *purK* housekeeping gene characterized epidemic isolates of VREF.

The enterococcal surface protein is a surface protein which is involved with interaction with a host cell. Said esp-protein contains a C-terminal cell-wall anchor domain, a central core containing tandem repeating units, and a globular N-terminal domain. It is hypothesized that the core region serves to maintain an elongated conformation to facilitate interaction of the N-terminal part of the protein with the host. Until the present invention, the *esp* gene was not detected in *E. faecium.*

The present invention discloses the unexpected fact, that the presence of an *esp* gene in an *E. faecium* bacterium indicates that said bacterium is epidemic. Non-epidemic bacteria do not comprise said *esp* gene. This is demonstrated in the examples and in table 1. With the teachings of the present invention, it is well possible to determine if an epidemic *E. faecium* bacterium has colonized an individual.

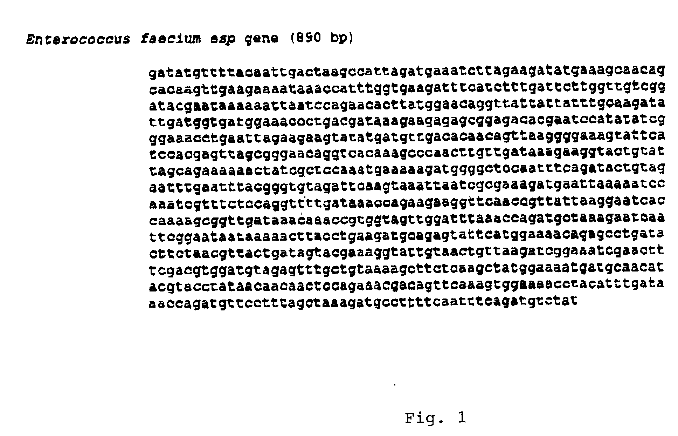

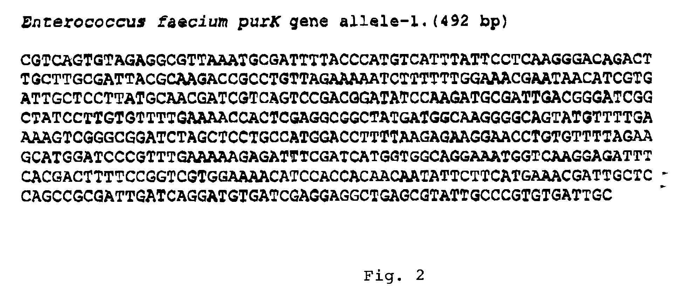

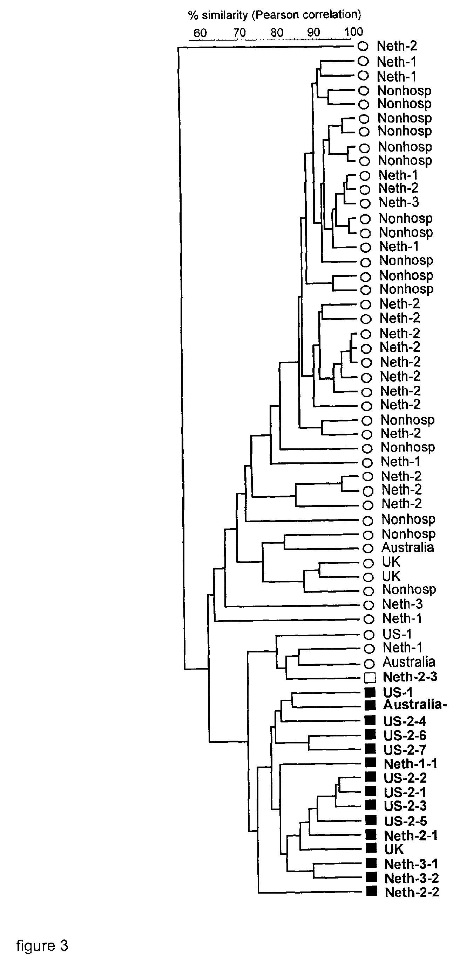

Additional analysis of the *E. faecium* housekeeping gene *purK* confirmed the existence of a distinct epidemic VREF genogroup. This gene is one of seven housekeeping genes selected for multi-locus sequence typing of *E*. *faecium* (manuscript in preparation). Twelve of 13 investigated epidemic clones had an identical *purK* sequence, designated allele-1, which differs in sequence (1 to 30 basepairs differences) from 23 out of 24 non-epidemic human isolates investigated (p <0.0001, Fischer's exact test) (Table) and from 17 selected isolates from farm animals (data not shown). Said *purK* sequence is depicted in figure 2.

The existence of an epidemic VREF subpopulation with specific genetic characteristics has important consequences for clinical practice. By an active infection control strategy, the prevalence of another feared multiresistant nosocomial pathogen, methicillin-resistant *Staphylococcus aureus* (MRSA), has been maintained extremely low in the Netherlands and the Scandinavian countries (37,38). This strategy includes surveillance of risk patients, mainly those transferred from hospitals abroad, separated care for MRSA-carriers and eradication of colonization (39). With a large community reservoir of VREF carriers, such a strategy seems inappropriate to control VREF. Our findings, however, demonstrate that only a subpopulation of VREF is associated with hospital outbreaks. Therefore, screening for the presence of the *esp* gene or a specific *purK* allele may provide a useful marker to direct infection control practices for the epidemic VREF subpopulation.

The present invention provides a method for determining whether an *E. faecium* bacterium is epidemic, comprising determining whether an epidemic *E. faecium* nucleic acid sequence and/or an expression product thereof is present in said bacterium, and/or associated with said bacterium. We have found that said epidemic *E. faecium* nucleic acid sequence comprises an *E. faecium esp* gene, and/or an epidemic *purK* housekeeping gene. By an epidemic *purK* housekeeping gene is meant herein a *purK* gene which is present in epidemic strains of *E. faecium.* For instance, said epidemic *purK* gene may comprise the sequence of figure 2. However, a person skilled in the art can think of many mutations which will retain the epidemic properties of said nucleic acid sequence in kind not necessarily in amount. For instance the third (wobble) position of each codon can be altered in a way that said codon is still encoding the same amino acid residue. Alternatively, a change in nucleic acid sequence may result in the substitution of one amino acid of the expression product into another. As long as the substituted amino acid residue has generally similar properties (like size and hydrophobicity), the overall function of said expression product will not be altered much and, hence, the function of the epidemic *purK* housekeeping gene will not be much altered.

Thus, in one aspect the invention provides a method of the invention, wherein said nucleic acid sequence comprises an *E. faecium esp* gene, and/or an epidemic *purK* housekeeping gene, or a functional equivalent thereof. Said nucleic acid sequences can be expressed in *E. faecium.* Thus in another aspect the invention provides a method of the invention wherein said expression product comprises an *esp* and/or epidemic *purK* expression product, or a functional part, derivative and/or analogue thereof.

By an epidemic *E. faecium* bacterium is meant herein an *E. faecium* bacterium which can spread rapidly and which is capable of causing disease in an individual. Said bacterium may comprise an intact cell with a bacterial genotype. Alternatively, said bacterium may be damaged, and/or artificially altered. For instance, a bacterium of the invention may lack a cell wall.

Associated with a bacterium is defined herein as being bound to (the exterior of) said bacterium. A molecule can for instance be bound to a bacterium by membrane proteins. Alternatively, a molecule can be bound to a bacterium by sticky interactions. Of course, a person skilled in the art can think of other ways of a molecule being associated with a bacterium.

An expression product is herein defined as a peptide which is encoded by a gene, preferably by an *E. faecium esp* or *purK* gene. Preferably, said expression product is a protein, for instance an *E. faecium esp* protein. Said expression product may be synthesized naturally, for instance in an *E. faecium* bacterium. Alternatively, said *expression product* may be synthesized artificially, according to the genetic code of a particular gene. Methods to artificially synthesize peptides are well known to the person skilled in the art. For instance, Pepscan may be used for this purpose.

The teachings of the present invention can particularly well be used for diagnostic purposes, especially to determine whether a patient suffers from an *E. faecium* related disease. Thus one embodiment of the invention provides a method for diagnosis of an epidemic *E. faecium* related disease in a patient, comprising:
- obtaining a sample from said patient, and
- detecting the presence of an *E. faecium esp* gene and/or an epidemic *purK* housekeeping gene or a functional equivalent thereof, and/or an expression product of any of said genes or a functional part, derivative and/or analogue of said product in said sample. Preferably, said sample is a blood sample.

A functional equivalent of a gene is defined herein as a part of said gene, at least 30 base pairs long, preferably at least 200 base pairs long, comprising at least one expression characteristic (in kind not necessarily in amount) as said gene. Preferably but not necessarily said part comprises the same expression characteristics.

A functional part of an expression product is defined herein as a part which has the same kind of properties in kind, not necessarily in amount. A functional derivative of an expression product is defined as an expression product which has been altered such that the properties of said molecule are essentially the same in kind, not necessarily in amount. A derivative can be provided in many ways, for instance through conservative amino acid substitution.

A person skilled in the art is well able to generate analogous compounds of a certain expression product. For instance, he can screen a peptide library for the same properties of said expression product (in kind, not necessarily in amount).

In one aspect the invention provides a method of the invention, wherein said *purK* housekeeping gene comprises a sequence as depicted in figure 2. However, as has been described before, a person skilled in the art can introduce a lot of mutations in said *purK* housekeeping gene without essentially altering the expression characteristics.

The presence of an *E. faecium esp* gene and/or an epidemic *purK* housekeeping gene can be determined by different methods known in the art. In a preferred embodiment, the presence of said *esp* or *purK* gene is determined by using a nucleic acid capable of specifically binding said *esp* or *purK* gene. Said nucleic acid may be used in an amplification reaction, to specifically amplify a nucleic acid molecule comprising said *esp* or *purK* gene. Said amplification reaction preferably comprise PCR and/or NASBA. Thus, one embodiment of the invention provides a method of the invention, wherein the presence of said *esp* gene and/or said epidemic purK gene is determined by using a nucleic acid molecule capable of specifically binding said *esp* or purK gene.

Alternatively, a pool of nucleic acid molecules may be amplified using generic primers. After amplification with said generic primers, the presence of a nucleic acid molecule comprising an *E. faecium esp* or epidemic *purK* gene may be determined by specific hybridization with a nucleic acid molecule capable of specifically binding said *esp* or epidemic *purK* gene. Said nucleic acid molecule may comprise a marker, for instance a fluorescent label, to distinguish nucleic acid molecules which are bound by said nucleic acid molecule from unbound nucleic acid molecules.

Figure 1 depicts an *E. faecium esp* gene sequence which we have determined (see examples). Thus, in a preferred embodiment, the invention provides a method of the invention wherein said *esp* gene comprises a sequence which is depicted in figure 1.

The *E. faecium esp* and/or epidemic *purK* gene are good indicators to determine whether an *E. faecium* strain is epidemic. When said *esp* and/or *purK* gene are expressed, the expression product is also a good indicator for said epidemic *E. faecium* strain. Thus, according to the invention, an *E. faecium esp* expression product is also useful for determination whether an *E. faecium* bacterium is epidemic. Said expression product can for instance be detected by a compound capable of specifically binding to said expression product. Said compound may be provided with a marker, for instance a fluorescent label. After a sample is incubated with those fluorescent binding molecules, and after unbound fluorescent molecules are washed away, fluorescence of said sample will reveal the presence of bound fluorescent molecules and, hence, the presence of an *esp* and/or *purK* expression product in said sample. Thus, one aspect of the invention provides a method of the invention, comprising providing a compound capable of specifically binding to said *esp* and/or purK expression product. Preferably, said compound comprises an antibody or a functional part, derivative and/or analogue thereof. As is known by a person skilled in the art, there are many other methods available in the art for detection of an expression product.

A functional part of an antibody is defined as a part which has the same kind of binding properties in kind, not necessarily in amount. Said functional part may for instance be a single chain antibody or a FAB fragment. A functional derivative of an antibody is defined as an antibody which has been altered such that the binding properties of said antibody are essentially the same in kind, not necessarily in amount. A derivative can be provided in many ways, for instance through conservative amino acid substitution.

A person skilled in the art is well able to generate analogous compounds of an antibody. This can for instance be done through screening of a peptide library. Such an analogue has essentially the same binding properties of said antibody in kind, not necessarily in amount.

In another aspect the invention provides an isolated or recombinant *E. faecium esp* expression product or a functional part, derivative and/or analogue thereof. Of course, the invention also provides an isolated or recombinant expression product of an epidemic *purK* housekeeping gene or a functional equivalent of said gene, or a functional part, derivative and/or analogue of said product. Preferably, said *purK* gene comprises a sequence as depicted in figure 2. As has already been described, an expression product of the invention can be obtained both naturally and artificially.

As the *esp* protein is a surface protein, patients carrying epidemic E. *faecium* will produce antibodies against said protein. The presence of said antibodies in a sample of said patient will thus indicate that said patient is infected with epidemic *E. faecium.* Of course, an isolated or recombinant *E. faecium esp* expression product or a functional part, derivative and/or analogue of the invention is particularly well suitable for determining the presence of said antibodies. For instance, said expression product can be coated in a well. After that, said well can be provided with a sample of a patient. Following a washing step, bound antibody can be detected by methods known in the art, for instance by provision of labeled goat anti-human antibodies. Thus, one embodiment of the invention provides a use of an expression product of the invention for determining whether a sample comprises a binding molecule capable of specifically binding an epidemic *E. faecium* bacterium, or a functional part, derivative and/or analogue thereof.

The invention also provides an isolated or recombinant antibody capable of specifically binding an expression product or a functional part, derivative and/or analogue of the invention, or a functional part, derivative and/or analogue of said antibody. An antibody of the invention is very well suited to determine the presence of a recombinant *E. faecium esp* expression product in a.patient, indicating an *E. faecium* related disease.

Now that an expression product and an antibody of the invention are provided, a person skilled in the art is well capable of constructing a nucleic acid molecule encoding said antibody. Methods to generate a nucleic acid molecule encoding a protein with a known amino-acid sequence are commonly known in the art. Alternatively, a person skilled in the art can isolate an *esp* and/or epidemic *purK* housekeeping gene from *E. faecium.* Said isolated genes can be modified by techniques known in the art. Thus, in one aspect the invention provides an isolated and/or recombinant nucleic acid molecule encoding *E. faecium esp* expression product or a functional part, derivative and/or analogue thereof. Preferably, said nucleic acid comprises a sequence as depicted in figure 1. Of course the invention also provides an isolated or recombinant nucleic acid molecule encoding an epidemic *purK* housekeeping gene or a functional equivalent thereof. In a preferred aspect, said nucleic acid molecule comprises a sequence as depicted in figure 2. In yet another aspect the invention provides an isolated and/or recombinant nucleic acid molecule encoding an antibody, functional part, derivative and/or analogue of the invention.

An *E. faecium esp* protein is involved with interaction with a host cell. Without being bound by theory it is thought that the capability of binding a host cell with an *esp* protein at least partly enhances the virulence of an esp-protein containing *E. faecium* bacterium. Therefore it is desirable to at least in part decrease the interaction between said *esp* protein and said host cell. Thus, another embodiment of the invention provides a method for treatment of an *E. faecium* related disease, comprising at least in part decreasing the attachment of an *E. faecium* bacterium to a host cell. Attachment is usually established through a certain receptor of the host cell. Therefore, another embodiment of the invention provides a method of the invention, comprising at least in part decreasing the attachment of said *E. faecium* bacterium to a receptor of said host cell.

It will be well understood that an antibody of the invention, directed against an *E. faecium esp* expression product, is very suitable for medical purposes. Thus, in one aspect the invention provides a use of an antibody according to the invention for the preparation of a medicament. Binding of said antibody to an *E. faecium* bacterium comprising an *esp* expression product will at least in part reduce attachment of said *E. faecium* bacterium to the host cell. If an *E. faecium* strain comprising an *esp* expression product is present in a patient, administration of an antibody of the invention will at least in part decrease colonization of said individual. This will at least partly decrease an *E. faecium* related disease in a patient. Thus, in yet another aspect, the invention provides a use of an antibody according to the invention for the treatment of an *E. faecium* related disease.

Another aspect of the invention provides a method of treatment of an epidemic *E. faecium* related disease in a patient, comprising administering to said patient an antibody of the invention. Preferably, said antibody is administered with a suitable carrier.

Considering the fact that *esp* amino acid sequences of various species comprise significant homology, the invention provides in yet another aspect an *E. faecium* esp-specific nucleic acid sequence capable of hybridising to at least a functional part of a nucleic acid sequence of to the invention. Preferably, said hybridisation is under stringent conditions. Through said hybridisation criterion, it is warranted that said nucleic acid sequence comprises similar expression characteristics (in kind not necessarily in amount), at least on mRNA level, as a nucleic acid sequence of the invention.

In another aspect the invention provides an *E. faecium esp* specific nucleic acid sequence, comprising at least 50 percent homology to a nucleic acid sequence of the invention. An *E. faecium esp* specific nucleic acid sequence is defined herein as a nucleic acid sequence, comprising at least 20 nucleotides, preferably at least 50 nucleotides, said sequence comprising a nucleic acid sequence corresponding to at least part of an *E. faecium esp* gene, or comprising a nucleic acid sequence which is complementary to a sequence corresponding to at least part of an *E. faecium esp* gene.

In a preferred aspect of the present invention, said *E. faecium esp* specific nucleic acid sequence comprises at least 60 percent homology to a nucleic acid of the invention. More preferably, said *E. faecium esp* specific nucleic acid sequence comprises at least 75 percent homology to a nucleic acid of the invention. In a most preferred aspect of the invention, said *E. faecium esp* specific nucleic acid sequence comprises at least 95 percent homology to a nucleic acid of the invention.

A nucleic acid and/or an expression product of the invention are particularly well suitable for diagnosis purposes. As has been described above, the presence of an epidemic *E. faecium* strain in a patient can be determined directly, by detecting the presence of an *E. faecium esp* gene and/or an epidemic *purK* housekeeping gene or a functional equivalent thereof, and/or an expression product of any of said genes. Additionally, the presence of an epidemic *E. faecium* strain in a patient can be determined indirectly by determining the presence in said patient of antibodies specifically directed towards an *esp* protein of said *E. faecium.* Thus, one embodiment of the invention provides a use of a nucleic acid sequence of the invention and/or an expression product thereof, for diagnosis of an *E. faecium* related disease in a patient.

In addition, an antibody of the invention is very well suited for use as a medicine. Therefore, another embodiment of the invention provides a pharmaceutical composition, comprising an antibody according to the invention. Of course, said pharmaceutical composition is very well suited for treatment of an *E. faecium* related disease. Thus, yet another embodiment of the invention provides a method of treatment of an epidemic *E. faecium* related disease in a patient, comprising administering to said patient a pharmaceutical composition according to the invention.

An antibody of the invention is capable of binding an *E. faecium* bacterium, preferably by binding an *esp* protein on the surface of said bacterium. Thus, an isolated *E. faecium* bacterium, bound by an antibody according to the invention, is also provided herewith. In addition to medical applications, specific binding of an antibody of the invention to *E. faecium* offers a major advantage for many different applications. For instance, *E. faecium* can now easily be isolated from a pool of microbes for further investigation.

Another embodiment of the invention provides an isolated cell, provided with a nucleic acid of the invention. This may be an isolated *E. faecium* bacterium. However, now that a nucleic acid of the invention is provided, it can be introduced into any cell, for instance for medical research purposes. A gene delivery vehicle comprising a nucleic acid of the invention is particularly well suited to introduce a nucleic acid of the invention into a certain target cell. Therefore, said gene delivery vehicle is also provided in the present invention. The introduction of a nucleic acid of the invention into a suitable target cell may for instance be used to generate (part of) an *esp* protein in high amounts. This obtained *esp* protein can be used to obtain antibodies directed against it. Additionally, a nucleic acid of the invention may be modified to obtain an *esp* protein with at least one modified property.

The present invention is further discussed in detail by the following examples. These examples are only intended for clarity purposes and do not limit the invention in any way.

### Examples

### Example 1. Determination of a genetically related epidemic VREF genogroup associated with hospital outbreaks

The aim of this study was to determine the genetic relationship between epidemic strains from the Netherlands and from other hospital outbreaks in Europe, the U.S. and Australia and non-epidemic *E. faecium* strains. We investigated 161 VREF that had been collected from hospital outbreaks in Europe and the U.S. (4, 27-31). Furthermore, four VREF isolated from hospitalized patients in Melbourne, Australia were analyzed (25). All isolates were genotyped by amplified fragment length polymorphism (AFLP) analysis (32). Based on this typing and on epidemiological linkage 120 isolates were considered epidemic and 45 non-epidemic (Table 1). The result (Figure 2) demonstrated the existence of a genetically related epidemic VREF genogroup associated with hospital outbreaks in three different continents, clearly distinct from non-epidemic hospital isolates, and from isolates recovered from European non-hospitalized persons which were genetically more diverse. Additional analysis of the *E. faecium* housekeeping gene *purK* confirmed the existence of a distinct epidemic VREF genogroup. This gene is one of seven housekeeping genes selected for multi-locus sequence typing of *E. faecium* (manuscript in preparation). *PurK* encodes a phosphoribosylaminoimidazole carboxylase ATPase subunit involved in purine biosynthesis. An 492 bp PCR fragment of the *purK* gene of representative epidemic clones from Europe, U.S. and Australia (see table 1) was sequenced using primers 5'-GCAGATTGGCACATTGAAAGT and 5'-TACATAAATCCCGCCTGTTTC/T. Furthermore, the *purK* sequence was determined of 20 non-epidemic hospital strains, 4 strains isolated from nonhospitalized persons, and 17 strains from farm animals. The PCR products were purified with a Qiagen PCR purification kit (Qiagen Inc., Hilden, Germany) according to the manufacturer's intsructions. Subsequently, the purified PCR products were sequenced directly with the ABI PRISM® Big Dye cycle sequencing ready reaction kit (Applied Biosystems, Foster City, Calif.) on an ABI PRISM® DNA analyzer (Applied Biosystems). Twelve of 13 investigated epidemic clones had an identical *purK* sequence (33), designated allele-1, which differs in sequence (1 to 30 basepairs differences) from 23 out of 24 non-epidemic human isolates investigated (p<0.0001, Fischer's exact test) (Table 1) and from 17 selected isolates from farm animals (data not shown).

### Example 2. Determination whether the esp gene is present in epidemic and non-epidemic VREF.

We determined the presence of the *esp* gene in all our VREF isolates. The presence of the *esp* gene was determined using a DNA probe based on the *E. faecalis esp* gene (Strain MMH594) generated by primers Esp11 (5'-TTGCTAATGCTAGTCCACGACC) and Esp 12 (5'-GCGTCAACACTTGCATTGCCGAA) (35). The *esp* probe was used to screen chromosomal digests of VREF by Southern Hybridization as described previously (11). An 890 bp internal *esp* fragment of 12 representative VREF isolates (Neth-1 (n=2), Neth-2 (n=2), UK (n=2), US-1 (n=2), US-2 (n=2), Australia (n=2)) was sequenced and compared to the previously described *E. faecalis esp* gene (35). To determine the DNA sequence of this internal fragment, a DNA fragment was amplified using primers Esp11 and Esp12 and purified with a Qiagen PCR purification kit (Qiagen Inc., Hilden, Germany) according to the manufacturer's instructions. Subsequently, the purified PCR product was sequenced directly with the PRISM® Big Dye cycle sequencing ready reaction kit (Applied Biosystems) on an ABI PRISM® DNA analyzer (Applied Biosystems). Finally, 890 bp of the sequence that was generated was compared with a homologous part of the *E. faecalis esp* sequence using MegAlign (DNASTAR, Inc, Madison, WI, U.S.).

The esp gene was detected in all blood isolates from US-1, UK, Neth-3, and Australia and in none of 16 stool isolates recovered from non-hospitalized persons. These preliminary data suggest that in *E. faecium* the presence of *esp* is associated with virulence. Furthermore, the *esp* gene was present in 15 of 16 epidemic clones and absent from all 29 non-epidemic hospital isolates, as well as from all 16 isolates of non-hospitalized persons (p<0.00001, Fischer's exact test) (Table 1). The *esp* gene was also absent in 98 VREF isolated from Dutch animals, including isolates from dogs (n=5), cats (n=2), pigs (n=22), chickens (n=28) and veal calves (n=41). Sequence analysis of an 890 bp internal *esp* fragment of representative isolates of UK, Neth-1, Neth-2, and Australia were identical and contained in this region of the *E. faecium esp* gene 25 bp mutations resulting in ten amino acid substitutions compared to the *E. faecalis esp* gene (36). One clone (US-1) contained 24 of these 25 mutations. Although only a small part of the *E. faecium esp* gene was sequenced, these findings demonstrate that the *E. faecium esp* gene in isolates causing hospital outbreaks in three continents is highly conserved and distinct from the *esp* gene in *E. faecalis.*

**TABLE 1 :**

| Frequency of the *esp* gene and the *purK* allele-1 among epidemic, non-epidemic and non-hospitalized *E. faecium* isolates | | | | | | | |
|---|---|---|---|---|---|---|---|
| Source, year | Epidemic^{a} | | | | Non-epidemic | | |
| | Clones | No of isolates | Esp positive | No *purK* allele-1/ no of isolates tested | No of isolates | Esp positive | No *purK* allele-1/ no of isolates tested |
| Neth-1, 2000 | 1 | 16 | 16 | 7/7 | 7 | 0 | 0/6 |
| Neth-2, 2000 | 1 | 20 | 20 | 10/10 | 14 | 0 | 0/11 |
| | 2 | 2 | 2 | 2/2 | | | |
| | 3 | 5 | 0 | 0/2 | | | |
| Neth-3, 1998-1999 | 1 | 25 | 25 | 5/5 | 2 | 0 | 0/2 |
| | 2 | 4 | 4 | 2/2 | | | |
| UK, 1992 | 1 | 11 | 11 | 1/1 | 3 | 0 | ND^{b} |
| US-1, 1992-1995 | 1 | 28 | 28 | 4/4 | 1 | 0 | ND |
| US-2, 1994-1995 | 1 | 1 | 1 | 1/1 | | | |
| | 2 | 1 | 1 | 1/1 | | | |
| | 3 | 1 | 1 | 1/1 | | | |
| | 4 | 1 | 1 | ND | | | |
| | 5 | 1 | 1 | ND | | | |
| | 6 | 1 | 1 | ND | | | |
| | 7 | 1 | 1 | 1/1 | | | |
| Australia, 1994-1998 | 1 | 2 | 2 | 1/1 | 2 | 0 | 1/1 |
| Non-hospitalized persons Neth, 1996-1998 | | | | | 16 | 0 | 0/4 |
| Total: | | 120 | 115 | 36/38 | 45 | 0 | 1/24 |

| | | | | | | | |
|---|---|---|---|---|---|---|---|
| ^{a} Isolates with AFLP patterns with greater than 90% similarity and with an epidemiological link, e.g. isolated from patients from the same ward and being admitted with overlapping time points were considered to belong to an epidemic clone. In three sets of strains, Neth-2, Neth-3, and US-2, different clones were identified and designated; Neth-2-1/2/3, Neth-3-1/2, and US-2 1/7. | | | | | | | |
| ^{b} ND = Not determined | | | | | | | |

### Brief description of the drawings

Figure 1: Sequence of the *E. faecium esp* gene.
Figure 2: Sequence of allele-1, an epidemic *purK* housekeeping gene.
Figure 3:
   Genetic relatedness of 60 vancomycin-resistant Enterococcus faecium strains as determined by AFLP. Strains were derived from seven hospital outbreaks in Europe (Neth-1, Neth-2, Neth-3, and UK) U.S., (US-1, US-2), Australia and from non-hospitalized persons in the Netherlands. Epidemic *esp* positive strains are marked in bold and by a filled square, while epidemic *esp* negative strains are marked in bold and by a open square. Only one representative isolate per epidemic clone is depicted. The non-epidemic hospital isolates and the strains isolated from non hospitalised individuals are marked by an open circle. The scale refers to % similarity, as determined by Pearson product moment correlation coefficient and unweighted pair group method with arithmetic averages using BioNumerics (version 1.5; Applied Maths, Kortrijk, Belgium) software.

### References

- 1.: M. L. Cohen, *Science* **257,** 1050 (1992).
- 2.: M. L. Cohen, *Nature* **406,** 762 (2000).
- 3.: Centers for Disease Control and Prevention, *Am. J. Infect. Control* **27,** 520 (1999).
- 4.: M. J. M. Bonten, M. K. Hayden, C. Nathan, T. W. Rice, R. A. Weinstein, *J. Infect. Dis.* **177,** 378 (1998).
- 5.: J. Bates, J. Z. Jordens, D. T. Griffiths, *J. Antimicrob. Chemother.* **34,** 507 (1994).
- 6.: I. Klare *et al., Microb. Drug Resist. **1**,* 265 (1995).
- 7.: F. M. Aarestrup *et al., Antimicrob. Agents Chemother.* **40,** 1938 (1996).
- 8.: L. A. Devriese *et al., Antimicrob. Agents Chemother.* **40,** 2285 (1996).
- 9.: A. E. van den Bogaard, P. Mertens, N. H. London, E. E. Stobberingh, *J. Antimicrob. Chemother.* **40,** 454 (1997).
- 10.: N. van den Braak *et al., J. Clin. Microbiol.* **36,** 1927 (1998).
- 11.: R. J. L. Willems *et al., Antimicrob. Agents Chemother.* **43,** 483 (1999).
- 12.: E. Stobberingh *et al., Antimicrob. Agents Chemother.* **43,** 2215 (1999).
- 13.: M. A. Schouten, A. Voss, J. A. A. Hoogkamp Korstanje, The European VRE Study Group, *Antimicrob. Agents Chemother.* **43,** 2542 (1999).
- 14.: P. Descheemaeker *et al., J. Infect. Dis.* **181,** 235 (2000).
- 15.: H. A. Kirst, D. G. Thompson, T. I. Nicas, *Antimicrob. Agents Chemother.* **42,** 1303 (1998).
- 16.: T. R. Friden *et al., Lancet* **342,** 76 (1993).
- 17.: R. Shekar, G. Chico, S. N. Bass, K. Strozewski, J. Biddle, *Clin. Infect. Dis.* **21,** 1511 (1995).
- 18.: J. J. Wade, *J. Hosp. Infect.* **30 Suppl,** 483 (1995).
- 19.: T. M. Coque, J. F. Tomayko, S. C. Ricke, P. C. Okhyusen, B. E. Murray, *Antimicrob. Agents Chemother.* **40,** 2605 (1996).
- 20.: B. E. Murray, *Am. J. Med.* **102,** 284 (1997).
- 21.: F. Bager, F. M. Aarestrup, M. Madsen, H. C. Wegener, *Microb. Drug Resist.* **5,** 53 (1999).
- 22.: I. Klare *et al., Microb. Drug Resist.* **5,** 45 (1999).
- 23.: A. Pantosti *et al., Lancet* **354,** 741 (1999).
- 24.: A. E. van den Bogaard, N. Bruinsma, E. E. Stobberingh, *J. Antimicrob. Chemother.* **46,** 146 (2000).
- 25.: J. M. Bell, J. C. Paton, J. Turnidge, *J. Clin. Microbiol.* **36,** 2187 (1998).
- 26.: O. Cars, S. Molstad, A. Melander, *Clin. Microbiol. Infect.* **6 (suppl. 1),** 216 (2000).
- 27.: J. Z. Jordens, J. Bates, D. T. Griffiths, *J. Antimicrob. Chemother.* **34,** 515 (1994).
- 28.: W. M. Dunne, W. Wang, *J. Clin. Microbiol.* **35,** 388 (1997).
- 29.: C. M. J. E. Vandenbroucke-Grauls *et al., Nederlands Tijdschrift Voor Medische Microbiologie* **8,** S28 (2000).
- 30.: E. M. Mascini *et al., Ned. Tijdschr. Geneeskd.* **144,** 2572 (2000).
- 31.: L. F. van der Steen *et al., Ned. Tijdschr. Geneeskd.* **144,** 2568 (2000).
- 32.: R. J. L. Willems *et al., J. Infect. Dis.* **182,** 816 (2000).
- 33.: L. M. Mundy, D. F. Sahm, M. Gilmore, *Clin. Microbiol. Rev.* **13,** 513 (2000).
- 34.: V. Shankar, A. S. Baghdayan, M. M. Huycke, G. Lindahl, M. S. Gilmore, *Infect. Immun.* **67,** 193 (1999).
- 35.: A. Voss, D. Milatovic, C. Wallrauch-Schwarz, V. T. Rosdahl, I. Braveny, *Eur. J. Clin. Microbiol. Infect. Dis.* **13,** 50 (1994).
- 36.: A. J. de Neeling *et al., J. Antimicrob. Chemother.* **41,** 93 (1998).
- 37.: C. M. Vandenbroucke-Grauls, *Infect. Control. Hosp. Epidemiol.* **17,** 512 (1996).

## Claims

1. A method for determining whether an *E. faecium* bacterium is epidemic, comprising determining whether an epidemic *E. faecium* nucleic acid sequence and/or an expression product thereof is present in said bacterium, and/or associated with said bacterium.

2. A method according to claim 1, wherein said nucleic acid sequence comprises an *E. faecium esp* gene, and/or an epidemic *purK* housekeeping gene, or a functional equivalent thereof.

3. A method according to claim 1 or 2, wherein said expression product comprises an *esp* and/or epidemic *purK* expression product, or a functional part, derivative and/or analogue thereof.

4. A method for diagnosis of an epidemic *E. faecium* related disease in a patient, comprising:
- obtaining a sample from said patient, and
- detecting the presence of an *E. faecium esp* gene and/or an epidemic *purK* housekeeping gene or a functional equivalent thereof, and/or an expression product of any of said genes or a functional part, derivative and/or analogue of said product in said sample.

5. A method according to any one of claims 2-4, wherein said *purK* housekeeping gene comprises a sequence as depicted in figure 2.

6. A method according to any one of claims 2-5, wherein the presence of said *esp* gene and/or said *purK* gene is determined by using a nucleic acid molecule capable of specifically binding said *esp* or *purK* gene.

7. A method according to any one of claims 2-6, wherein said *esp* gene comprises a sequence which is depicted in figure 1.

8. A method according to any one of claims 2-7, comprising providing a compound capable of specifically binding to said *esp* and/or *purK* expression product.

9. A method according to claim 8, wherein said compound comprises an antibody or a functional part, derivative and/or analogue thereof.

10. An isolated or recombinant *E. faecium esp* expression product or a functional part, derivative and/or analogue thereof.

11. Use of an expression product according to claim 10 for determining whether a sample comprises a binding molecule capable of specifically binding an epidemic *E. faecium* bacterium, or a functional part, derivative and/or analogue thereof.

12. An isolated or recombinant expression product of an epidemic *purK* housekeeping gene or a functional equivalent of said gene, or a functional part, derivative and/or analogue of said product.

13. A product according to claim 12, wherein said *purK* gene comprises a sequence as depicted in figure 2.

14. An isolated or recombinant antibody capable of specifically binding an expression product or a functional part, derivative and/or analogue according to claim 10 or 12, or a functional part, derivative and/or analogue of said antibody.

15. An isolated and/or recombinant nucleic acid molecule encoding *E. faecium esp* expression product or a functional part or derivative thereof.

16. A nucleic acid molecule according to claim 15, comprising a sequence as depicted in figure 1.

17. An isolated or recombinant nucleic acid molecule encoding an epidemic *purK* housekeeping gene or a functional equivalent thereof.

18. A nucleic acid molecule according to claim 17, comprising a sequence as depicted in figure 2.

19. An isolated and/or recombinant nucleic acid molecule encoding an antibody or functional part, derivative and/or analogue according to claim 14.

20. A method for treatment of an *E. faecium* related disease, comprising at least in part decreasing the attachment of an *E. faecium* bacterium to a host cell.

21. A method according to claim 20, comprising at least in part decreasing the attachment of said *E. faecium* bacterium to a receptor of said host cell.

22. Use of an antibody or functional part, derivative and/or analogue according to claim 14, for the preparation of a medicament.

23. Use of an antibody or functional part, derivative and/or analogue according to claim 14, for at least in part treatment of an *E. faecium* related disease.

24. An *E. faecium esp* specific nucleic acid sequence capable of hybridising to at least a functional part of a nucleic acid sequence according to claim 15 or 16.

25. A nucleic acid sequence according to claim 24, wherein said hybridisation is under stringent conditions.

26. An *E. faecium esp* specific nucleic acid sequence, comprising at least 50 percent homology to a nucleic acid sequence according to claim 15 or 16.

27. An *E. faecium esp* specific nucleic acid sequence, comprising at least 75 percent homology to a nucleic acid sequence according to claim 15 or 16.

28. An *E. faecium esp* specific nucleic acid sequence, comprising at least 95 percent homology to a nucleic acid sequence according to claim 15 or 16.

29. A pharmaceutical composition, comprising an antibody according to claim 14.

30. An isolated *E. faecium* bacterium, bound by an antibody according to claim 14.

31. A cell, provided with a nucleic acid according to anyone of claims 15-19 or 24-28.

32. A gene delivery vehicle comprising a nucleic acid according to anyone of claims 15-19 or 24-28.

33. A method of treatment of an epidemic *E. faecium* related disease in a patient, comprising administering to said patient a pharmaceutical composition according to claim 29.

34. Use of a nucleic acid sequence according to anyone of claims 15-19 or 24-28, and/or an expression product thereof, for diagnosis of an *E. faecium* related disease in a patient.
